# EUROPEAN PATENT SPECIFICATION

(11) **EP 1 314 743 B1**
(45) Date of publication and mention of the grant of the patent: **15.10.2014**
(21) Application number: 02025633.5
(22) Date of filing: 19.11.2002
(51) Int. Cl.: A61K 8/73, A61Q 19/00, C08B 31/00, C08B 35/00, A23L 1/0522

(54) **Low amylose and adipate crosslinked tapioca starch**
Tapioka-Stärke mit niedrigen Gehalt an Amylose und vernetzt mit Adipinsäure
Amidon de tapioca à faible teneur en amylose et réticulé par de l'acide adipique

(30) Priority: 21.11.2001 US 339615 P; 22.10.2002 US 278215
(43) Date of publication of application: 28.05.2003
(73) Proprietor: CORN Products Development Inc., Westchester, IL 60154 (US)
(72) Inventor: Jeffcoat, Roger, Bridgewater, New Jersey 08873 (US); Hanchett, Douglas J., Mine Hill, New Jersey 07801 (US); Tayal, Akash, South Somerset, New Jersey 08873 (US)
(74) Representative: Held, Stephan

(56) References cited:
- EP-A- 0 338 121
- EP-A- 0 358 444
- EP-A- 1 249 496
- US-A- 2 461 139
- US-A- 4 207 355
- US-A- 4 229 489

## Description

The present invention relates to the use of an acetylated low amylose tapioca distarch adipate wherein the starch has an amylose content of less thann 10% by weight in paper products, food products, pharmaceutical products, nutritional products, personal care products, detergents and biodegradable foamed products, as well as compositions comprising tapioca starch, wherein at least a portion of the starch in the composition is substituted by said acetylated low amylose tapioca distarch adipate.

Starch, in general, contains two types of polymers, amylose which is essentially linear and amylopectin which is branched. Tapioca starch, obtained from cassava, has been known for many years. Regular tapioca starch typically has an amylose content of 20-23% by weight; the balance being amylopectin. It has been used in a variety of industrial applications, including papermaking, food products, pharmaceuticals, and personal care products. It is often used instead of the more traditional corn starches-for a variety of reasons, including low protein content and bland flavor relative to corn.

To meet the demanding needs of the food industry, starch is often modified by numerous techniques known in the industry to change the behavioral characteristics from that of the unmodified starch. Although native tapioca starch and its derivatives are adequate for many applications, it would be desirable to have a tapioca starch which is low in amylose and would thus have different properties which would result in improved functionality when used in industrial applications.

Low amylose starches are known in the art. Low amylose, or waxy, corn has been used for many years for a variety of applications. Low amylose rice starch is gaining importance, particularly in Asia. Low amylose potato starch has also been disclosed in WO 92/11376.

Starches which are crosslinked by using a bi- or poly-functional chemical reagent that is able to react with two or more different hydroxyl groups on the same or different starch molecule are also well known in the art. This type of chemical modification controls granular swelling and produces a starch that is process tolerant; that is, one that can tolerate high temperature, high shear and/or acidic conditions.

Distarch adipates, prepared by crosslinking with mixed adipic and acetic anhydride reagents, and the methods of producing them are also known in the art. The mixed anhydride reagent used creates organic ester linkages that are relatively stable under many typical processing conditions. See for example U.S. Patent No. 2,461,139 (Caldwell, 8 February, 1949).

When an aqueous dispersion of native tapioca starch is heated, the starch granules begin to swell, and the dispersion develops a short, salve-like texture which is important in imparting palatability and thickening in systems. However, during the process of cooking native starches, this textural state rapidly changes to an elastic, rubbery state in which the swollen granules rupture. Minor variations in cooking time, temperature, and concentration as well as shear and pH are sufficient to effect this transformation. Crosslinking acts to strengthen the granules by reinforcing the hydrogen bonds which are responsible for holding the granules intact and thus are used to overcome the extreme sensitivity of the swollen starch granules to handling and processing conditions.

Surprisingly, it has now been determined that adipic/acetic crosslinked low amylose tapioca starches have superior process tolerance compared to similarly crosslinked regular tapioca starches as well as similarly crosslinked regular and waxy maize starches.
The present invention relates to the use of an acetylated low amylose tapioca distarch adipate wherein the starch has an amylose content of less than 10% by weight in paper products, food products, pharmaceutical products, nutritional products, personal care products, detergents and biodegradable foamed products and compositions containing at least a portion of such acetylated low amylose tapioca distarch adipate.
Figure 1 depicts the relative molecular weight distribution by GPC of the regular and low amylose tapioca starches.
Figure 2 depicts the Brabender viscosity of distarch adipates prepared from regular and low amylose tapioca starches, corn starch and waxy maize starch.
The present invention relates to the use of an acetylated low amylose tapioca distarch adipate wherein the starch has an amylose content of less than 10% by weight in paper products, food products, pharmaceutical products, nutritional products, personal care products, detergents and biodegradable foamed products and compositions containing at least a portion of such acetylated low amylose tapioca distarch adipate.

Low amylose starch, as used herein, is intended to mean a starch or flour which has an amylose content substantially lower than that of regular tapioca starch, particularly less than 10%, more particularly less than 5%, most particularly less than 3% amylose by weight.

Low amylose tapioca starch may be obtained by the method of U.S. Application Number 09/832,626 or is derived from low amylose cassava plants which may be found in nature, obtained by standard breeding and crossbreeding techniques, or obtained by translocation, inversion, transformation or any other method of gene or chromosome engineering to include variations thereof, whereby the properties of the starch of this invention are obtained. In addition, starch extracted from a plant grown from artificial mutations and variations of the above generic composition which may be produced by known standard methods of mutation breeding is also applicable herein.
The substantially pure starch may be extracted from the root of a low amylose cassava plant. Extraction may be by any method known in the art, including but not limited to pulverizing the root and separating the starch from the remaining components by water extraction.

The native low amylose tapioca starch is crosslinked using mixed adipic and acetic anhydride reagents. Such reagents and the crosslinking reaction are well known in the art for making distarch adipates using other native starches. For example, the low amylose tapioca distarch adipates may be prepared by reacting starch in an aqueous slurry with an adipic/acetic mixed anhydride reagent. The bound acetyl of the resultant starch may be adjusted by one skilled in the art to any level necessary for the amount of stability desired, particularly in the range of up to 2.5% bound acetyl. The amount of adipic/acetic mixed anhydride used in the reaction may also be adjusted by one skilled in the art to provide the desired inhibition effect (degree of crosslinking) in the resultant starch. Typically, the amount of mixed anhydride used is less than 1%.

The resultant low amylose tapioca distarch adipates have properties and functionality which are unique and desirable in many applications. The low amylose tapioca distarch adipates have a peak viscosity which is higher than that of regular tapioca, corn or waxy maize starches which have been modified in the same manner. Under acid conditions, viscosity breakdown is minimal and occurs slowly after the peak viscosity is reached. This is indicative of process tolerance in low pH systems such as a cherry pie filling or fruit prep. Yet, the viscosity onset and rise to peak of the low amylose tapioca distarch adipates are similar to that of regular tapioca distarch adipates. Further, the low amylose distarch adipates reach peak viscosity quickly. Such viscosity profiles are highly desirous in many industrial applications.

The presently disclosed low amylose tapioca distarch adipates may be further modified to enhance their properties and functionality. In particular, the starch diadipates may be physically modified, such as by thermal inhibition described in WO 95/04082 (published February 9, 1995) or by shear.

The low amylose tapioca distarch adipates may also be pregelatinized. Exemplary processes for preparing pregelatinized starches are disclosed in U.S. 4,280,851 (Pitchon, et al.), U.S. 4,465,702 (Eastman, et al.), U.S. 5,037,929 (Rajagopalan), U.S. 5,131,953 (Kasica, et al.), and U.S. 5,149,799 (Rubens). Conventional procedures for pregelatinizing starch are well known to those skilled in the art and described in such articles as Chapter XXII- "Production and Use of Pregelatinized Starch", Starch: Chemistry and Technology, Vol. III - Industrial Aspects, R.L. Whistler and E.F. Paschall, Editors, Academic Press, New York 1967.

The low amylose tapioca starch adipates may be purified by any method known in the art to remove off-flavors and colors that are native to the starch or created during starch modification processes. Purification processes preferred for treating the present starches are disclosed in U.S. Serial No. 07/832,838 filed February 7, 1992, by Kasica, et al. Alkali washing techniques, for starches intended for use in either granular or pregelatinized form, are also useful and described in the family of patents represented by U.S. 5,187,272 (Bertalan, et al.). Purification can either be done to the native low amylose tapioca starch prior to crosslinking or to the distarch adipate.

One skilled in the art is capable of using any single or combination of modifications known in the art in order to obtain the desired starch properties and functionality. These modifications are well known in the art and the resulting starch properties and functionality will vary depending, *inter alia,* on the type of modification employed, the degree of modification, and the reaction conditions.

The low amylose tapioca distarch adipates of the present invention may be used in a variety of industrial applications, including without limitation paper products, food products, pharmaceutical and nutritional products, personal care products and other industrial products.

Paper products is intended to include, without limitation, paper, paperboard, linerboard, corrugating, cardboard, bags, and envelopes.

Food products is intended to mean any edible product and includes, without limitation, cereals, breads and bread products, cheese and imitation cheese products, condiments, confectioneries, dressings including pourable dressings and spoonable dressings, pie fillings including fruit and cream fillings, sauces, including white sauces and dairy-based sauces such as cheese sauces, gravies, imitation and lite syrups, puddings, custards, yogurts, sour creams, pastas, beverages including dairy-based beverages, glazes, soups and baby food.

Pharmaceutical and nutritional products is intended to include pharmaceutical excipients, tablets including effervescent tablets, dusting starches and powders, and prebiotics products.

Personal care products is intended to include without limitation deodorants and antiperspirants, hair fixatives including sprays, gels, mousses, lotions and pomades, soaps and cleansers, makeup including eye shadow, powders, foundations, and blushers, shampoos and conditioners, and mouthwashes, breath fresheners and toothpastes.

Other industrial products intend to include without limitation detergents, and biodegradable foamed products including loosefill, sheets and shapes.

The low amylose tapioca distarch adipates may generally be used at any desired level, the amount being dependent upon the functionality to be obtained. In general, the starch will be used in an amount of from 1% to 95%, particularly from 5% to 60%, more particularly in an amount of 10% to 40% by weight of the product.

The following examples are presented to further illustrate and explain the present invention and should not be taken as limiting in any regard. All percents used are on a weight/weight basis.

In the examples below, the tapioca samples used are as follows: Tapioca = regular tapioca starch commercially available from National Starch and Chemical Company (Bridgewater, NJ, USA).
LATS = low amylose tapioca starches genetically produced by introducing the GBSS gene in the antisense mode and using FEC from which cassava plants are regenerated.
Corn = regular corn starch commercially available from commercially available from National Starch and Chemical Company (Bridgewater, NJ, USA).
Waxy = waxy maize starch commercially available from National Starch and Chemical Company (Bridgewater, NJ, USA).

### Example 1 - Amylose content

a. Amylose content was determined by Potentiometric titration. Approximately 0.5 g of a starch sample was heated in 10 ml of concentrated calcium chloride (about 30% by weight) to 95°C for 30 minutes. The sample was cooled to room temperature, diluted with 5 ml of a 2.5% uranyl acetate solution, mixed well, and centrifuged for 5 minutes at 2000 rpm. The sample was then filtered to give a clear solution.
The starch concentration was determined polarimetrically using a 1 cm Polarimetric cell. An aliquot of the sample (normally 5 ml) was then directly titrated with a standardized 0.01 N iodine solution while recording the potential using a platinum electrode with a KCI reference electrode. The amount of iodine needed to reach the inflection point was measured directly as bound iodine. The amount of amylose was calculated by assuming 1.0 gram of amylose will bind with 200 miligrams of iodine.
The results of the Potentiometric titration are showed in Table I.

**Table I**

| Base starch | Amylose Content (%) |
|---|---|
| Tapioca | 20% |
| LATS | 2.7% |

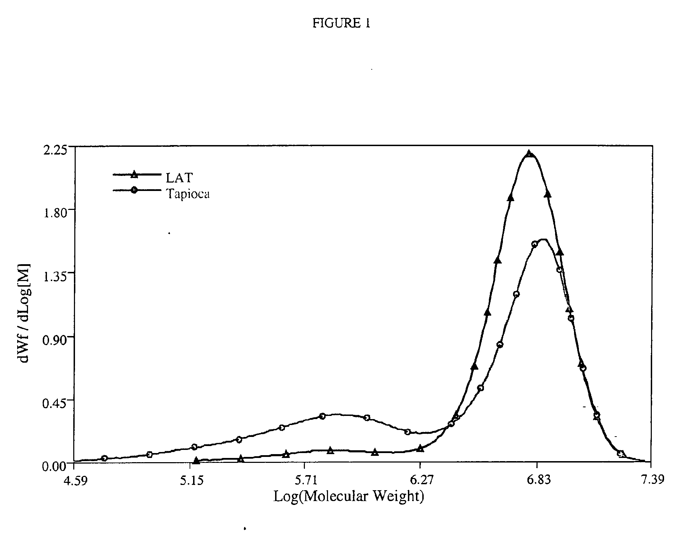

As can be determined from Table I, the low amylose tapioca starches contain significantly less amylose than the regular tapioca starches.
b. The amylose contents were checked by gel permeation Chromatograph (GPC). Samples were prepared for analysis by slurrying 4 to 8 mg of starch in 4 g of dimethylsulfoxide (DMSO) containing 5mM sodium nitrate and heating to 100°C for 2 hours. The sample was filtered if necessary, and injected (300 µl) into a GPC 150C chromatograph (Waters Corporation, Amherst, MA). The Gel Permeation chromatograph utilized 4 columns (guard column, 10⁵, 10³, 10² micron (nominal) pore size columns, all from Polymer Laboratories, Amherst, MA). The mobile phase was dimethyl sulfoxide containing 5 mM of sodium nitrate. The instrument was operated at a temperature of 80°C and a flow rate of 0.7 ml/minute was used. The columns were calibrated with pullulan standards (Showa Denko K.K., Japan) ranging in molecular weight from 5800 to 850,000. Figure 1 shows the relative molecular weight distribution by GPC of the regular and low amylose tapioca starches. As can be determined from the figure, the low amylose tapioca starch has significantly more amylopectin as seen by the peak at a relative log (molecular weight) of about 6.83. Further, this is the sole main peak. In contrast, the regular starch shows an additional amylose peak at a relative log (molecular weight) of about 6.

### Example 2 - Preparation of the Distarch adipates

a. 100 grams of low amylose tapioca starch was slurried in 150 grams water and brought to a temperature of 27°C. The pH of the slurry was then adjusted to 8.0 using a 3% NaOH solution. The reagent was mixed in a separate flask using 3.9%(wt/wt) acetic anhydride and 0.9%(wt/wt) of a 1:9 adipic:acetic mixed anhydride based on the weight of the starch. This mixture was then added to the starch slurry at a controlled rate during which the pH was kept constant at pH 8.0 until the reaction was complete. The pH was then adjusted to 6.0 using a dilute HCI solution and the starch was washed and dried.
b. The method of Example 2a was repeated using tapioca starch.
c. The method of Example 2a was repeated using corn starch.
d. The method of Example 2a was repeated using waxy maize starch.

### Example 3 - Viscosity

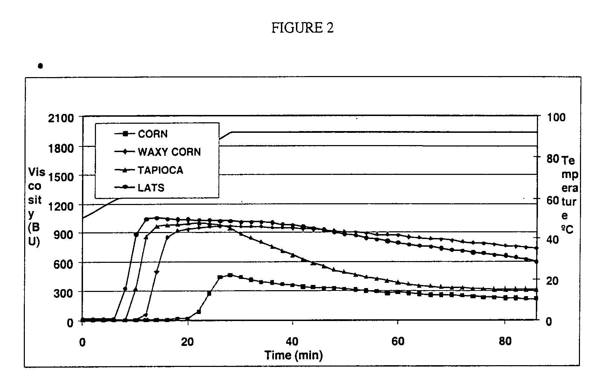

The viscosity of the starches of Example 2 was measured using a Visco/amylo/graph, Model VA-1A (C.W. Brabender Instrument Co., Hackensack, NJ, USA07606). A slurry of 5% starch on a dry weight basis was prepared and controlled to pH3 using a citric acid/trisodium citrate buffer solution. The total charge weight of 460grams was heated from 50°C to 92°C at a rate of 1.5°C per minute. The slurry was then held at 92°C for 30 minutes. The hot viscosity was measured while heating the paste in the Visco/amylo/graph. The results are shown in Figure 2.

As can be seen from Figure 2, the low amylose tapioca distarch adipate has a peak viscosity which is higher than that of regular tapioca, corn or waxy maize starches which have been modified in the same manner. Under acid conditions, viscosity breakdown is minimal and occurs slowly after the peak viscosity is reached. This is indicative of process tolerance in low pH systems. The viscosity onset and rise to peak of the low amylose tapioca distarch adipate is similar to that of regular tapioca distarch adipate, but the low amylose distarch adipates reach peak viscosity quickly.

## Claims

1. Use of an acetylated low amylose tapioca distarch adipate wherein the starch has an amylose content of less than 10 % by weight in paper products, food products, pharmaceutical products, nutritional products, personal care products, detergents, and biodegradable foamed products.

2. Use according to claim 1, wherein the starch has an amylose content of less than 5 % by weight.

3. Use according to claim 1, wherein the starch has an amylose content of less than 3 % by weight.

4. Use according to any one of claim 1 to 3, wherein the product comprises the acetylated low amylose tapioca distarch adipate in an amount of from 1 % to 95 % by weight.

5. A composition comprising tapioca starch, wherein at least a portion of the starch in the composition is substituted by an acetylated low amylose tapioca distarch adipate, wherein the starch has an amylose content of less than 10 %.

6. A composition according to claim 5 comprising the acetylated low amylose tapioca distarch adipate of claim 1, wherein the composition is selected from the group consisting of paper products, food products, pharmaceutical products, nutritional products, personal care products, detergents, and biodegradable foamed products.

## Patentansprüche

1. Verwendung eines acetylierten, amylosearmen Tapiokadistärkeadipats,
wobei die Stärke einen Amylosegehalt von weniger als 10 Gew.-% aufweist, bei Papierprodukten, Lebensmittelprodukten, pharmazeutischen Produkten, Ernährungsprodukten, Körperpflegeprodukten, Reinigungsmitteln und biologisch abbaubaren geschäumten Produkten.

2. Verwendung nach Anspruch 1,
wobei die Stärke einen Amylosegehalt von weniger als 5 Gew.-% aufweist.

3. Verwendung nach Anspruch 1,
wobei die Stärke einen Amylosegehalt von weniger als 3 Gew. % aufweist.

4. Verwendung nach einem der Ansprüche 1 bis 3,
wobei das Produkt das acetylierte, amylosearme Tapiokadistärkeadipat in einer Menge von 1 bis 95 Gew.-% aufweist.

5. Zusammensetzung, die Tapiokastärke aufweist,
wobei zumindest ein Teil der Stärke in der Zusammensetzung durch ein acetyliertes, amylosearmes Tapiokadistärkeadipat ersetzt ist, wobei die Stärke einen Amylosegehalt von weniger als 10 % aufweist.

6. Zusammensetzung nach Anspruch 5, die acetyliertes, amylosearmes Tapiokadistärkeadipat nach Anspruch 1 aufweist,
wobei die Zusammensetzung aus der Gruppe ausgewählt ist, die aus Papierprodukten, Lebensmittelprodukten, pharmazeutischen Produkten, Ernährungsprodukten, Körperpflegeprodukten, Reinigungsmitteln und biologisch abbaubaren geschäumten Produkten besteht.

## Revendications

1. Utilisation d'un adipate de diamidon de tapioca acétylé à faible teneur en amylose, dans laquelle l'amidon présente une teneur en amylose inférieure à 10 % en poids dans des produits papetiers, des produits alimentaires, des produits pharmaceutiques, des produits nutritionnels, des produits de soins personnels, des détergents et des produits foisonnés biodégradables.

2. Utilisation selon la revendication 1, dans laquelle l'amidon présente une teneur en amylose inférieure à 5 % en poids.

3. Utilisation selon la revendication 1, dans laquelle l'amidon présente une teneur en amylose inférieure à 3 % en poids.

4. Utilisation selon l'une quelconque des revendications 1 à 3, dans laquelle le produit comprend un adipate de diamidon de tapioca acétylé à faible teneur en amylose en une quantité comprise entre 1 % et 95 % en poids.

5. Composition comprenant de l'amidon de tapioca, dans laquelle une partie au moins de l'amidon dans la composition est substituée par un adipate de diamidon de tapioca acétylé à faible teneur en amylose, dans laquelle l'amidon présente une teneur en amylose inférieure à 10 %.

6. Composition selon la revendication 5 comprenant un adipate de diamidon de tapioca acétylé à faible teneur en amylose la revendication 1, dans laquelle la composition est sélectionnée dans le groupe constitué par des produits papetiers, des produits alimentaires, des produits pharmaceutiques, des produits nutritionnels, des produits de soins personnels, des détergents et des produits foisonnés biodégradables.
